# EUROPEAN PATENT SPECIFICATION

(11) **EP 2 294 228 B1**
(45) Date of publication and mention of the grant of the patent: **12.02.2020**
(21) Application number: 09779622.1
(22) Date of filing: 03.06.2009
(51) Int. Cl.: C13B 10/08, C13B 10/02, C13K 1/02, C12P 7/10, D21C 3/20, D21C 3/22, D21C 3/04

(54) **METHOD FOR PRETREATING PLANT STARTING MATERIAL FOR THE PRODUCTION, FROM SACCHARIFEROUS AND LIGNOCELLULOSIC RESOURCES, OF BIOETHANOL AND/OR OF SUGAR**
VERFAHREN ZUR VORBEHANDLUNG EINES PFLANZENSTARTMATERIALS ZUR HERSTELLUNG VON BIOETHANOL UND/ODER ZUCKER AUS ZUCKERPRODUZIERENDEN ODER LIGNOZELLULOSEHALTIGEN RESSOURCEN
ROCÉDÉ POUR PRÉTRAITER UN MATÉRIAU DE DÉPART DE PLANTE POUR LA PRODUCTION, À PARTIR DE RESSOURCES SACCHARIFÈRES ET LIGNOCELLULOSIQUES, DE BIOÉTHANOL ET/OU DE SUCRE

(30) Priority: 23.06.2008 FR 0854121
(43) Date of publication of application: 16.03.2011
(73) Proprietor: Compagnie Industrielle De La Matiere Vegetale CIMV, 92300 Levallois Perret (FR)
(72) Inventor: BENJELLOUN MLAYAH, Bouchra, F-31450 Pompertuzat (FR); DELMAS, Michel, F-31320 Auzeville-Tolosane (FR); LEVASSEUR, Gérard, F-44230 Saint-Sébastien-sur-Loire (FR); SCHOLASTIQUE, Thierry, F-33680 Lacanau Océan (FR)
(74) Representative: Gevers & Orès
(86) International application number: PCT/EP2009/056806
(87) International publication number: WO 2010/006840

(56) References cited:
- WO-A-00/68494
- WO-A-2008/028183
- WO-A-2008/029163
- FR-A- 2 605 644
- FR-A- 2 885 371
- US-A- 3 266 933
- US-A- 6 139 683
- US-B1- 6 258 175
- MARINA O S DIAS ET AL: "Evaluation of Energy Demand During Bioethanol Production from Sugarcane and Sugarcane Bagasse-Computer Based Scenario Approach", 18TH EUROPEAN SYMPOSIUM ON COMPUTER AIDED PROCESS ENGINEERING, 2008, JUNE 1 - 4; LYON, FRANCE, ELSEVIER, US , 1 January 2008 (2008-01-01), pages 1-6, XP008159428, Retrieved from the Internet: URL:http://www.nt.ntnu.no

## Description

### TECHNICAL FIELD OF THE INVENTION

The present invention relates to the exploitation of biomass, and in particular the non-dietary use of agricultural products. The invention relates in particular to the production of bioethanol from plant material.

The exploitation of agricultural plant productions, other than in the form of food products that can be virtually directly consumed by humans or animals, requires a very large amount of industrial investment, in particular for the production of plants for converting the plant starting material.

Thus, for the purpose of producing sugar from sacchariferous resources, such as sugar beet or sugar cane, it is necessary to have a sugar refinery, the actual annual use of which, depending on the plant starting material used, ranges from 2400 to 3600 hours/year.

Outside these periods of production, which correspond to periods of agricultural production of the plant starting material concerned, the plants are unused.

Many methods of production have been optimized in order in particular to reduce production costs and to increase industrial exploitation, for example in the context of the production of sugar and alcohol from sugar cane or beet.

Whether it involves the use of sugar cane or beet, the operation of which the purpose is to extract therefrom, by diffusion, a sugar juice (for the production of sugar and of bioethanol) requires heated water to be circulated counter-current to the plant material.

In the same way, during the extraction of the sugar juice from sugar cane, a stream of hot water is injected in order to facilitate the extraction of the sugar juice.

An example thereof is given in document FR-A-2.605.644, which proposes improvements to the principle of extraction by diffusion from beet chips so as, at the end of this pretreatment, to produce a sugar juice which is conveyed into sugar manufacture, the chips then being subjected to a second extraction by diffusion so as to produce a sugar juice intended for fermentation, for subsequent treatment thereof in a distillery.

However, such optimized methods do not provide any solution to the problem of the low annual use of sugar manufacture and distillery plants.

Moreover, the processes for production of a sugar juice from sugar cane or beet, which have just been mentioned above, result in the production of residues which, in the case of beet, are called draff or pulps, which are wet fibrous residues that leave the diffusion phase and that usually contain less than 10% of solids, and drying of which can in particular make them a possible source of feed for cattle, which constitutes the principal use of these residues.

FR 2 885 371 relates to similar installation for pretreatment of lignocellulosic material pretreatment (hydrolysis for cellulose, hemicellulose and lignin). MARINA O S DIAS ET AL: "Evaluation of Energy Demand During Bioethanol Production from Sugarcane and Sugarcane Bagasse-Computer Based Scenario Approach", 18TH EUROPEAN SYMPOSIUM ON COMPUTER AIDED PROCESS ENGINEERING, 2008, JUNE 1 - 4; LYON, FRANCE, ELSEVIER, US, pages 1-6 discloses an installation for lignocellulosic material (pretreatment, hydrolysis for cellulose, hemicellulose and lignin) and sugar extraction (sugar cane to bagasse). The processes share the infrastructure (such as fermentation and distillation units) and equipment costs are diminished. In the case of the production of a sugar juice from sugar cane, the residue, called bagasse, is a fibrous residue which is, at the current time, used as a fuel by the sugar production company itself, or else in power stations, in which the bagasse constitutes the starting material to be burnt (bagasse-fired/coal-fired power station). Such a use as starting material in a power station means, given the seasonal sugar cane harvesting campaigns, that there must be considerable means for storing the bagasse.

The proprietor of the present application has, moreover, designed and developed a method for pretreating a lignocellulosic plant starting material for obtaining a pretreated material which can be hydrolysed and fermented for the production of bioethanol.

This method makes it possible, under particularly economical and efficient conditions, to produce bioethanol industrially from lignocellulosic resources constituted, for example, by whole plants or parts of these plants (stems, barks, etc.) or co-products from industrial procedures of which the purpose is production (wheat, rice, barley straw, sugar cane bagasse, sugar sorghum bagasse, etc.).

This pretreatment method is described and represented in French Patent Application No. 08 50458 filed on 25 January 2008.

In the context of its research and development studies, the proprietor has been able to note that, at the current time, there is no solution which makes it possible to reduce the overall industrial investment for the production of sugar and of bioethanol from the two main categories of plant starting material that sacchariferous resources and lignocellulosic resources constitute.

### SUMMARY OF THE INVENTION

With this objective, the invention proposes a method for pretreating plant starting material for the purpose of producing bioethanol and sugar comprising the steps consisting in:
- providing a common chamber for pretreating the plant material, comprising:
   -- at least one downstream inlet for introducing plant material to be pretreated into the common pretreatment chamber;
   -- at least one downstream outlet for discharging the pretreated plant material from the common pretreatment chamber;
   -- means for circulating the plant material from upstream to downstream;
   -- means for bringing the plant material into contact with a pretreatment liquid which circulates overall, from downstream to upstream, in the opposite direction to the direction of circulation of the plant material inside said common pretreatment chamber;
   -- and means for recovering, on the one hand, the solid phase and, on the other hand, the liquid phase containing in particular at least a part of the pretreatment liquid;
- during one period, introducing into the common pretreatment chamber a lignocellulosic plant starting material to be treated (for example, straw), said pretreatment of which is aimed at separating the cellulose, the hemicelluloses and the lignins contained in this lignocellulosic plant starting material so as to obtain a pretreated plant material that can be hydrolysed and fermented for the production of bioethanol;
- during another period, introducing into the common pretreatment chamber, a sacchariferous plant starting material to be pretreated (for example sugar cane or beet), said pretreatment of which is aimed at extracting therefrom, by diffusion, a sugar juice for the production of sugar and of bioethanol.

By virtue of the method according to the invention, it is thus possible, by means of the same industrial plant, and as a function of the seasonal availability of one category or the other of plant starting material, i.e. of sacchariferous resources or of lignocellulosic resources, to use the corresponding plant fulltime, or virtually fulltime, i.e. about 8000 hours/year.

According to other features of the invention:
- during said one period, the pretreatment liquid is a mixture containing formic acid and water at a temperature of between 95°C and 110°C;
- during said other period, the pretreatment liquid is water at a temperature above 70°C;
- said pretreatment stage is carried out at atmospheric pressure, or at a slightly reduced pressure;
- during said one period, the plant material to be pretreated is a part of the solid phase recovered at the end of said other period.

### BRIEF DESCRIPTION OF THE FIGURE

Other features and advantages of the invention will emerge on reading the detailed description which follows and, for the understanding of which, reference will be made to the attached drawing in which:
- the figure is a schematic representation of an exemplary embodiment of a pretreatment plant in accordance with the teachings of the invention, and given by way of nonlimiting example.

### DETAILED DESCRIPTION OF THE FIGURE

In the description which follows, all the identical, similar or analogous elements and components will be denoted by the same references.

The terms "longitudinal", "vertical" and "transversal" will be used with reference to the trihedron L, V, T indicated on the figure.

The upstream-downstream orientation will also be used for the longitudinal circulation of the plant material inside the common chamber, from right to left, taking the figure into consideration, along the L axis.

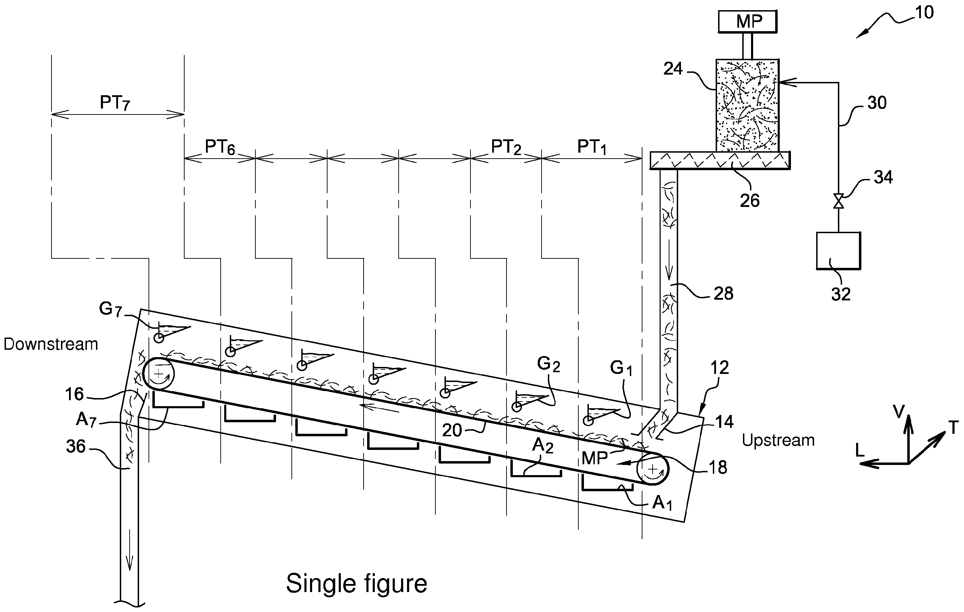

The plant 10 illustrated schematically in Figure 1 comprises a pretreatment chamber 12 which is in the general shape of a right-angled parallelepipedal chamber that is oriented longitudinally and substantially horizontal, for example with a slight slope from downstream to upstream as illustrated in Figure 1.

The pretreatment chamber 12 is sealed so as to prevent any dissipation of acid vapour into the atmosphere, when acids are used in the context of the method according to the invention.

The chamber comprises an upstream inlet 14 for feeding starting material and a downstream outlet 16 for expelling or discharging the pretreated starting material from the chamber 12.

The pressure inside the pretreatment chamber 12 is atmospheric pressure.

Inside the chamber 12 is a motorized conveyor 18, the belt 20 of which, in the upper part, moves from upstream to downstream, from right to left, and receives, in the area of its upstream end, the plant starting material MP to be pretreated, entering the pretreatment chamber 12 via the inlet 14.

The conveyor 18 belt 20 thus makes it possible to circulate the starting material from upstream to downstream inside the chamber 12, at a constant speed or at a speed controlled by drive and control means, and also by means, not represented, for controlling the speed at which the starting material MP is moved.

The belt 20 extends over a given transverse width and it is, for example, constituted of a corrugated sheet made of materials resistant to acid mixtures.

The starting material MP is preferably distributed as uniformly as possible, by means not represented, over the entire width of the belt 20 of the conveyor 18.

The belt 20 is arranged in the pretreatment chamber 12 in such a way that a liquid which reaches the upper face of the upper belt 20 can flow, for example laterally, on either side of the longitudinal edges of the belt, and/or, by way of a variant, through the belt 20, which is then perforated for this purpose.

The plant 10 comprises a hopper 24 for feeding the pretreatment chamber 12 with starting material MP.

The hopper 24 is herein connected to the inlet 14 via a screw 26 for propelling the starting material into a pipe 28 connected to the inlet 14.

As illustrated in Figure 1, the hopper 24 can be connected, via piping 30, to a reservoir 32 containing a mixture of organic acids in order, depending on the category of plant starting material to be treated, to carry out in the hopper 24 a first pretreatment of the starting material MP by pre-impregnation of the starting material. The flow rate for feeding the hopper 24 with pre-impregnation acid mixture can be controlled via a solenoid valve 34.

When the starting material MP leaves the upper belt 20 of the conveyor 18, it drops by gravity into the outlet 16 and it is expelled via an expulsion pipe 36, this part, which is recovered at the end of the pretreatment, constituting the solid phase within the meaning of the invention.

In addition to the pretreatment chamber 12 and the means for feeding said chamber with starting material MP, the plant 10 comprises, here successively from upstream to downstream, a series of n pretreatment stations PTi, with i between 1 and n.

In the example illustrated in Figure 1, the number of pretreatment stations is equal to seven.

Thus, the first pretreatment station upstream is the station PT1, while the last station downstream is the station PT7.

All the components of a station PTi will be denoted by the same references with the suffix "i".

The function of each pretreatment station PTi is to temporarily place together or bring into contact the starting material MP and a pretreatment liquid.

From the figure, the various consecutive pretreatment stations are defined by mixed vertical lines.

Each pretreatment station PTi comprises, arranged vertically above the upper belt 20 transporting the starting material MP, means for sprinkling the starting material with pretreatment liquid, by gravity.

By way of nonlimiting example, the means for sprinkling the starting material MP are here, at each station, constituted of a bucket Gi which, on the figure, is illustrated in the resting and filling position and which is capable of tipping on its lower horizontal axis so as to tip its content out vertically, and substantially over the entire transverse width of the belt 20, onto the starting material MP located on the upper belt 20 substantially perpendicular to the bucket Gi.

By way of a variant, which is not represented, the means for sprinkling the starting material at each station may be constituted of one or more ramps for sprinkling or spraying the starting material by gravity, always in such a way as to guarantee as homogeneous a distribution of the pretreatment liquid as possible.

Each station PTi also comprises means for recovering the pretreatment liquid after this liquid has passed through the starting material MP, and has then flowed laterally over either side of the conveyor belt 20 and/or passed through the belt if the latter is perforated or has an openwork design for this purpose, with perforations that are sufficiently small in size to allow only the liquid to be recovered to pass through. This part that is recovered at the end of the pretreatment constitutes the liquid phase within the meaning of the invention.

The means for collecting the liquid phase after it has passed through the starting material MP are here constituted, at each station, of a collecting trough Ai which extends transversely over the entire width of the pretreatment chamber 12 and, longitudinally, substantially over the entire length of a pretreatment station PTi.

A more complete and more detailed plant is described structurally and in operational terms in Patent Application FR-A-2.885.371 in the name of the proprietor, which concerns a plant for implementing a method for producing paper pulp, lignins and sugars.

The pretreatment plant which has just been described is merely one example of the various possible designs in the context of the implementation of the method according to the invention.

In accordance with the teachings of the invention, during one period PL, or campaign, the plant starting material to be pretreated, which is introduced into the pretreatment chamber 12, is a lignocellulosic plant starting material, whereas, during another period PS, the plant starting material to be pretreated, introduced into the pretreatment chamber 12, is a sacchariferous plant starting material.

Thus, the pretreatment chamber 12 is a chamber "common" to the two types of treatment associated with the two categories of plant starting material mentioned above.

Similarly, depending on each of the plant starting materials to be pretreated, it may also be possible to make the entire plant common, if the plant starting materials allow it, i.e. to make the means for feeding and expelling the starting material common.

When the pretreatment stage carried out in the common chamber 12 concerns lignocellulosic plant starting material, the treatment liquid is a mixture containing, at least in part one or more acids and the common chamber 12 is, to this effect, a sealed chamber in order to prevent any leaking of acids to the outside.

When the plant starting material is a sacchariferous resource, acids are not normally used, and the pretreatment liquid is heated water, for example heated to a temperature of approximately 70°C or above.

When the pretreatment liquid is a mixture containing acid, and in particular formic acid, and water, said liquid is used at a temperature of between 95°C and 110°C.

The pretreatment operations are preferably carried out at atmospheric pressure, or at a slightly reduced pressure.

The fact that a common treatment chamber is used means that it is possible to switch very easily, with a very short interruption of the operating of the plant (for example of the order of one or two days) from one treatment period to the other treatment period, depending on the seasons and/or on the availabilities of the lignocellulosic or sacchariferous plant starting materials.

When the pretreated plant starting material is of the sacchariferous type, the solid phase recovered at the end of the pretreatment for the purpose of producing the sugar juice can, completely or partially and in particular depending on the plant used, be re-used as lignocellulosic-type plant starting material inside the common chamber 12 so as to undergo a pretreatment step for obtaining a pretreated plant material that can be hydrolysed and fermented for the production of bioethanol.

Thus, by means of the same plant, and for example in the case of the use of sugar cane, the bagasse - instead of being stored with a view to its use as a fuel - is re-used and exploited in the form of a lignocellulosic plant starting material.

Such an additional exploitation of the solid phase derived from the pretreatment of a sacchariferous starting material is possible, whatever the plant used, and the yield thereof depends on the lignocellulose content of the solid phase recovered.

Of course, for example in the case of sugar cane, a part of the bagasse may, in a known manner, be used directly in the context of the plant, in particular as fuel for heating the liquids.

## Claims

1. Method for pretreating plant starting material for the purpose of producing bioethanol and sugar, comprising the steps consisting in:
- providing a common chamber (12) for pretreatment of the plant material, comprising:
-- at least one upstream inlet (14) for introducing plant material (MP) to be pretreated into the common pretreatment chamber;
-- at least one downstream outlet (16) for discharging the pretreated plant material from the common pretreatment chamber (12);
-- means (20) for circulating the plant material from upstream to downstream;
-- means (Gi) for bringing the plant material into contact with a pretreatment liquid which circulates overall, from downstream to upstream, in the opposite direction to the direction of circulation of the plant material inside said common pretreatment chamber;
-- and means for recovering, on the one hand, the solid phase and, on the other hand, the liquid phase containing in particular at least a part of the pretreatment liquid;
- during one period (PL), introducing into the common pretreatment chamber (12), a lignocellulosic plant starting material to be pretreated, said pretreatment of which is aimed at separating the cellulose, the hemicelluloses and the lignins contained in said lignocellulosic plant starting material so as to obtain a pretreated plant material that can be hydrolysed and fermented for the production of bioethanol;
- during another period (PS), introducing into the common pretreatment chamber, a sacchariferous plant starting material to be pretreated, said pretreatment of which is aimed at extracting therefrom, by diffusion, a sugar juice for the production of sugar and of bioethanol.

2. Method according to Claim 1, **characterized in that**, during said one period (PL), the pretreatment liquid is a mixture containing formic acid and water at a temperature of between 95°C and 110°C.

3. Method according to either one of the preceding claims, **characterized in that**, during said other period (PS), the pretreatment liquid is water at a temperature above 70°C.

4. Method according to any one of the preceding claims, **characterized in that** said pretreatment step is carried out at atmospheric pressure, or at a slightly reduced pressure.

5. Method according to the preceding claim, **characterized in that**, during said one period (PL), the plant material to be pretreated is a part of the solid phase recovered at the end of said other period.

## Patentansprüche

1. Verfahren zum Vorbehandeln von pflanzlichem Ausgangsmaterial zum Zweck des Produzierens von Bioethanol und Zucker, umfassend die Schritte, bestehend aus:
- Bereitstellen einer gemeinsamen Kammer (12) zur Vorbehandlung des pflanzlichen Materials, umfassend:
-- mindestens einen vorgelagerten Einlass (14) zum Einbringen von vorzubehandelndem pflanzlichem Material (MP) in die gemeinsame Vorbehandlungskammer;
-- mindestens einen nachgelagerten Auslass (16) zum Ausstoßen des vorbehandelten pflanzlichen Materials aus der gemeinsamen Vorbehandlungskammer (12);
-- Mittel (20) zum Zirkulieren des pflanzlichen Materials von vorgelagert zu nachgelagert;
-- Mittel (Gi) zum In-Kontakt-Bringen des pflanzlichen Materials mit einer Vorbehandlungsflüssigkeit, die insgesamt in der entgegengesetzten Richtung der Richtung des Zirkulierens des pflanzlichen Materials innerhalb der gemeinsamen Vorbehandlungskammer von nachgelagert zu vorgelagert zirkuliert;
-- und Mittel zum Wiedergewinnen von einerseits der festen Phase und andererseits der flüssigen Phase, die insbesondere mindestens einen Teil der Vorbehandlungsflüssigkeit enthält;
- während eines Zeitraums (PL), Einbringen eines vorzubehandelnden lignocellulosischen pflanzlichen Ausgangsmaterials in die gemeinsame Vorbehandlungskammer (12), wobei die Vorbehandlung davon auf Trennen der in dem lignocellulosischen pflanzlichen Ausgangsmaterial enthaltenen Cellulose, der Hemicellulosen und der Lignine abzielt, um so ein vorbehandeltes pflanzliches Material zu erhalten, das für die Produktion von Bioethanol hydrolysiert und fermentiert werden kann;
- während eines anderen Zeitraums (PS), Einbringen eines vorzubehandelnden zuckerhaltigen pflanzlichen Ausgangsmaterials in die gemeinsame Vorbehandlungskammer, wobei die Vorbehandlung davon auf Extrahieren eines Zuckersafts durch Diffusion daraus für die Produktion von Zucker und von Bioethanol abzielt.

2. Verfahren nach Anspruch 1, **dadurch gekennzeichnet, dass** während des einen Zeitraums (PL) die Vorbehandlungsflüssigkeit eine Mischung ist, die Ameisensäure und Wasser bei einer Temperatur zwischen 95 °C und 110 °C enthält.

3. Verfahren nach einem der vorstehenden Ansprüche, **dadurch gekennzeichnet, dass** während des weiteren Zeitraums (PS) die Vorbehandlungsflüssigkeit Wasser bei einer Temperatur über 70 °C ist.

4. Verfahren nach einem der vorstehenden Ansprüche, **dadurch gekennzeichnet, dass** der Vorbehandlungsschritt bei Atmosphärendruck oder bei einem leicht verringerten Druck ausgeführt wird.

5. Verfahren nach dem vorstehenden Anspruch, **dadurch gekennzeichnet, dass** während des einen Zeitraums (PL) das vorzubehandelnde pflanzliche Material ein Teil von der am Ende des weiteren Zeitraums wiedergewonnenen festen Phase ist.

## Revendications

1. Procédé de prétraitement de matière première végétale pour produire du bioéthanol et du sucre, comprenant les étapes consistant à :
- fournir une chambre commune (12) pour le prétraitement de la matière végétale comprenant :
--au moins une entrée amont (14) pour introduire la matière végétale (MP) à prétraiter dans la chambre de prétraitement commune ;
-- au moins une sortie aval (16) pour décharger la matière végétale prétraitée hors de la chambre de prétraitement commune (12) ;
-- des moyens (20) pour faire circuler la matière végétale d'amont en aval ;
-- des moyens (Gi) pour mettre la matière végétale en contact avec un liquide de prétraitement qui circule globalement, de l'aval vers l'amont, dans le sens inverse du sens de circulation de la matière végétale à l'intérieur de ladite chambre de prétraitement commune ;
-- et des moyens pour récupérer, d'une part, la phase solide et, d'autre part, la phase liquide contenant notamment au moins une partie du liquide de prétraitement ;
- pendant une période (PL), introduire dans la chambre de prétraitement commune (12) une matière première végétale lignocellulosique à prétraiter, dont ledit prétraitement vise à séparer la cellulose, les hémicelluloses et les lignines contenues dans ladite matière première végétale lignocellulosique de manière à obtenir une matière végétale prétraitée qui peut être hydrolysée et être fermentée pour la production de bioéthanol ;
- durant une autre période (PS), introduire dans la chambre de prétraitement commune une matière première végétale saccharifère à prétraiter, dont ledit prétraitement vise à en extraire, par diffusion, un jus sucré pour la production de sucre et de bioéthanol.

2. Procédé selon la revendication 1, **caractérisé en ce que**, pendant ladite une période (PL), le liquide de prétraitement est un mélange contenant de l'acide formique et de l'eau à une température comprise entre 95 °C et 110 °C.

3. Procédé selon l'une quelconque des revendications précédentes, **caractérisé en ce que** durant ladite autre période (PS), le liquide de prétraitement est de l'eau à une température supérieure à 70 °C.

4. Procédé selon l'une quelconque des revendications précédentes, **caractérisé en ce que** ladite étape de prétraitement est effectuée à pression atmosphérique, ou à une pression légèrement réduite.

5. Procédé selon la revendication précédente, **caractérisé en ce que**, durant ladite une période (PL), la matière végétale à prétraiter est une partie de la phase solide récupérée à l'issue de ladite autre période.
